# EUROPEAN PATENT APPLICATION

(11) **EP 1 997 521 A1**
(43) Date of publication of application: **03.12.2008**
(21) Application number: 07738822.1
(22) Date of filing: 16.03.2007
(51) Int. Cl.: A61L 27/00, A61C 8/00, A61F 2/28, A61F 2/30

(54) **MEDICAL MATERIAL**

(30) Priority: 17.03.2006 JP 2006075154; 17.03.2006 JP 2006075153
(71) Applicant: HI-LEX Corporation, Takarazuka-shi, Hyogo 665-0845 (JP)
(72) Inventor: KUBOKI, Yoshinori, Sapporo-shi, Hokkaido 063-0038 (JP); SEKI, Yasuo, Takarazuka-shi, Hyogo 665-0845 (JP); SHIOTA, Hiroyuki, Takarazuka-shi, Hyogo 665-0845 (JP)
(74) Representative: Bailey, David Martin
(86) International application number: PCT/JP2007/055377
(87) International publication number: WO 2007/108411

(57) **Abstract**

A medical material 10 used for an artificial tooth root (Figure 1b) or an artificial bone (Figure 1c). The medical material comprises a support 11 composed of titanium web and an outer layer 12 composed of bioabsorbable calcium phosphate compound formed in the periphery thereof.

## Description

### Field of the Invention

This invention relates to a medical material, particularly to a medical material used for bone fixation fixtures, or substitutional bones for artificial tooth root implants, artificial joint implants, bone plates etc.

### Background Arts

Patent Document 1: Japanese Published Patent Application No.2004-67547
Patent Document 2: Japanese Utility Model Patent NO. H7-41467
Patent Document 3: Examined Utility Model Application Publication No.2002-345948
Patent Document 4: Japanese Patent No.3554349
Patent Document 5: Japanese Published Patent Application No.2005-95584
Patent Document 6: WIPO Publication No.WO2003/35128
Patent Document 7: Japanese Published Patent Application No.2005-111255
Patent Document 8: Japanese Published Patent Application No.2005-112716
Patent Document 9: WIPO Publication No.WO2003/70291
Patent Document 10: Japanese Patent No.3646167

Titanium metal is used as a medical material in various ways such as implant materials, substitutional bones including artificial tooth roots, artificial joints. It is because the titanium metal has an excellent property of being little in foreign body reactions in a living body compared with other metals. Further, it has no magnetism, it is high in strength, and it is light weight etc.
At the same time, it is known that ceramics composed of calcium phosphate compound has excellent biocompatibility. Among them, apatite hydroxide, α-tricalcium phosphate, β-tricalcium phosphate are known to bond directly with bone tissues etc. And it is known that these are used as the medical material for prosthetic appliance of bone of artificial tooth roots, artificial joints etc. Further, α-tricalcium phosphate, β-tricalcium phosphate are known to be absorbed in a living body with time, and known to be substituted by an autogenous bone.

Various medical materials, which use ceramics composed of titanium metal and calcium phosphate compounds are disclosed.
In Patent Document 1, a biological hard tissue-induced scaffold material is disclosed. This scaffold material is composed of a titanium fiber or a titanium base alloy fiber whose aspect ratio is 20 or more, and it is intertwined to be formed in layers. Further, it said that the fiber surface of this scaffold material is coated with calcium phosphate compound such as apatite hydroxide.

In Patent Document 2, a composite prosthetic appliance of bone having a mesh material made of titanium, and hardened body of calcium phosphate system formed surrounding the mesh material; and a composite prosthetic appliance of bone having mesh material made of ceramics fibers, and hardened body of calcium phosphate system formed surrounding the mesh material are disclosed. Here, as the ceramic fiber, alumina fibers, carbon fibers etc. are cited. And as the hardened body of calcium phosphate system, α-tricalcium phosphate and tetracalcium phosphate etc. are cited. It is said that these composite prosthetic appliances of bone have strong mechanical strength and do not fall into pieces if broken. Therefore, it is described that it is effective for anchoring when bone tissues are induced.

In Patent Document 3, a composite sintered compact having a titanium metal, and a porous calcium phosphate system compound layer formed on its surface is disclosed.

In Patent Document 4, a compound material in which a composite membrane of titanium oxide and zinc oxide is formed on the surface of calcium phosphate system ceramics is disclosed.
In Patent Document 5, a biological implant material in which a coating layer whose main component is titanium or titanium alloy is formed on the surface of calcium phosphate system ceramics is disclosed.

In Patent Document 6, a composite biomaterial composed of hydroxyapatite, collagen, and alginate is disclosed. This composite biomaterial has micro porous structure in which the c axis of hydroxyapatite (apatite hydroxide) is oriented so as to be along collagen fibers. It is said that this composite biomaterial has excellent biocompatibility and bone inductivity.

In Patent Document 7, 8, a calcium phosphate compound material in which an apatite core forming agent composed of calcium phosphate being fixed on the surface of a substrate composed of ceramics is disclosed.

### Disclosure of invention

### Problems to be resolved by the invention

The implant material composed of metal nonwoven fabric of titanium etc. as shown in Patent Document 1 has strength because it is made of metal. However since it only physical coupling is made between the material and induced bone tissues etc., two to three months are necessary for bone tissues to infiltrate sufficiently.
In contrast, ceramics of the calcium phosphate system couples with the tissues in short period of time. It is because the ceramic couples chemically with the bone tissues. However it does not have sufficient strength. For example, in the material composed only of ceramics like disclosed in Patent Document 6, the mechanical strength to keep in a living body is not sufficient, when it is actually used as a prosthetic appliance of bone or an artificial tooth root.

The first purpose of this invention is to provide a new medical material whose strength is high, and whose inducing period of the cells is short. This new medical material is made by composing a metal fiber composed of titanium or titanium alloy and calcium phosphate system ceramics into layers.

The second purpose of this invention is to provide a new medical material whose strength is high, and whose inducing period of the cells is short. This new medical material is made by composing ceramics into layers.

### Means of solving the problems

The medical material of this invention (claim 1) comprises a metal nonwoven fabric layer composed of a titanium metal fiber whose one side or diameter is 100µm or less, and a ceramic layer composed of calcium phosphate compound provided adjacently to the metal nonwoven fabric layer. Here, the one side refers to the one side of a metal fiber whose cross section is polygon.
In such medical material, it is preferable that the ceramic layer is composed of bioabsorbable calcium phosphate compound (claim 2), or the ceramic layer is composed of non-bioabsorbable calcium phosphate compound (claim 3).
Further, it is preferable that the metal nonwoven fabric layer is formed to be in a tube shape or a column shape, and the ceramic layer is provided in the periphery thereof (claim 4), or the ceramic layer is formed to be in a tube shape or a column shape, and the metal nonwoven fabric layer is provided in the periphery thereof (claim 5).

The second aspect of the medical material of this invention comprises a first layer composed of ceramics of which the porosity is 0.1-10 % (low porosity), and a second layer provided adjacently to the first layer composed of ceramics of which the porosity is 11-80 % (high porosity). Further, the difference of the porosity of the first layer and the second layer is 10-80 points (claim 6). In such medical material, it is preferable that at least two or more of the first layers are equipped (claim 7). And, as the ceramics of the first layer and the second layer, it is preferable to use bioabsorbable ceramics and non-bioabsorbable ceramics (claim 8, 9). Further, as the ceramics of first layer, bioabsorbable ceramics or non-bioabsorbable ceramics may be used, and as the ceramics of the second layer, non-bioabsorbable ceramics or bioabsorbable ceramics may be used (claim 10).

As such medical material, the first layer may be formed to be in a tube shape or a column shape and the second layer is provided in the periphery thereof (claim 11). Moreover, first layer may be formed to be in a lattice shape, and the second layer is provided in the space between, so as to fill in the clearance thereof (claim 12).

The third aspect of this invention comprises a tube shape or a column shape metal rod and an outer layer composed of bioabsorbable ceramics or non-bioabsorbable ceramics provided in the periphery thereof (claim 13).

### Effect of the invention

The medical material of this invention comprises a metal nonwoven fabric layer composed of a titanium metal fiber whose one side or diameter is 100µm or less, and a ceramic layer composed of calcium phosphate compound provided adjacently to the metal nonwoven fabric layer. Therefore, when this medical material is implanted in a living body, cells are induced into the medical material, and the chemical coupling and physical coupling of the cells with the ceramics layer, and physical coupling of the cells with the metal nonwoven fabric layer are obtained. Resultantly the firm bonding of the cell and the medical material are achieved. And, since the metal nonwoven fabric layer is composed of titanium metal fiber of the diameter 100 µm or less, cells are regenerated in a structure similar to the living body (claim 1).

In such medical material where ceramics layer is bioabosorbable calcium phosphate compound (claim 2), since the ceramics layer will be replaced with living cells after the treatment, only the metal nonwoven fabric layer remains. And the cells will be regenerated in a structure similar to the living body. Moreover, when a sufficient period has passed over after the treatment, the treated area can be easily taken out by repeat surgery comparatively, since only the metal nonwoven fabric layer remains.
Moreover, when the ceramic layer is bio-nonabsorbable calcium phosphate compound (claim 3), since the induced cells couples with the ceramic layer and the metal nonwoven fabric layer, the medical material will be firmly coupled in the living body.

When the metal nonwoven fabric layer is formed to be in a tube shape or a column shape, and the ceramic layer is provided in the periphery thereof (claim 4), or the ceramic layer is formed to be in a tube shape or a column shape, and the metal nonwoven fabric layer is provided in the periphery thereof (claim 5), these medical material can be used as an implant material of artificial tooth root or an artificial bone.

The second aspect of this invention comprises a first layer composed of ceramics of which the porosity is 0.1-10 % (low porosity), and a second layer provided adjacently to the first layer composed of ceramics of which the porosity is 11-80 % (high porosity). The low porosity first layer improves the whole strength of the medical material. The high porosity second layer having a geometric structure which cells most preferably infiltrate, induces cells. Resultantly second layer and the cells firmly bonds by the physical coupling. Moreover, since the difference of porosity between the first layer and the second layer is 10-80 points, shear after cells being fixed by the infiltration will be prevented. In other words, the second embodiment of medical material of this invention can keep strong strength on average from the start when medical material is inserted in the living body to the end when the induced cells and the material are firmly fixed. Therefore, it is particularly preferably to be used as the material for inducing osteoblasts such as artificial tooth root, artificial bone, prosthetic appliance of bone (claim 6).

When bioabsorbable ceramics is used as the first layer and/or second layer of such medical materials (claim 8), the inducing period of cells is comparatively short. And, it physically bonds first with the infiltrating cells and finally absorbed into or by the cells. Further, when non-bioabsorbable ceramics is used as the first layer and/or the second layer (claim 9), though the inducing period of cells may becomes slow, the strength of the medical material can be obtained.

As such medical material, in the case where the first layer is formed to be in a tube shape or a column shape, and the second layer is provided in the periphery thereof (claim 11), since the second layer having the geometrical structure which cells preferably infiltrates, is in the outside, it can induce cells sequentially to the second layer and to the first layer in a comparatively short period.

The third aspect of medical material of this invention comprises a tube shape or a column shape metal rod and an outer layer composed of bioabsorbable ceramics or non-bioabsorbable ceramics provided in the periphery thereof. Therefore, the whole strength of the medical material is strong. This medical material can preferably be used as an artificial tooth root, an artificial bone, and a prosthetic appliance of bone (claim 13).

### Best mode for carrying out the invention

The medical material of this invention is described using drawings.
Figures 1a, b are cross sections showing embodiments of medical material of this invention respectively, Figure 1c is a cross section when the medical material is used as an artificial tooth root, Figure 1d is a cross section when the medical material is used as an artificial bone.
Figures 2a, b, c are cross sections showing the other embodiments of medical material of this invention, Figure 2d is a cross section when the medical material is used as an artificial tooth root, Figure 2e is a cross section when the medical material is used as an artificial bone.
Figures 3a, b are cross sections showing the other embodiments of medical material of this invention, Figure 3c is a cross section when the medical material is used as an artificial tooth root, Figure 3d is a cross section when the medical material is used as an artificial bone.
Figures 4a, b are cross sections showing further the other embodiments of medical material of this invention, Figure 4c is a cross section when the medical material is used as an artificial tooth root, Figure 4d is a cross section when the medical material is used as an artificial bone.
Figure 5 is a cross section when the medical material is used as an artificial joint.
Figure 6a is a cross section when the medical material of this invention is used as a bone plate, Figure 6b is a cross section when the medical material of this invention is used as a prosthetic appliance of bone.
Figure 7 is an electrophotograph showing the medical material of this invention.
Figures 8a, b are cross sections showing an embodiments of medical material of this invention, Figure 8c is a cross section when the medical material is used as an artificial tooth root, Figure 8d is a cross section when the medical material is used as an artificial bone.
Figures 9a, b, c are cross sections showing the other embodiments of medical material of this invention, Figure 9d is a cross section when the medical material is used as an artificial tooth root, Figure 9e is a cross section when the medical material is used as an artificial bone.
Figures 10a, b, c, d are cross sections showing further the other embodiments of medical material of this invention.
Figures 11a, b are cross sections showing further the other embodiments of medical material of this invention, Figure 11c is a cross section when the medical material is used as an artificial tooth root, Figure 11d is a cross section when the medical material is used as an artificial bone.
Figure 12 is a cross section when the medical material is used as an artificial joint.
Figure 13a is a cross section when the medical material of this invention is used as a bone plate, Figure 6b is a cross section when the medical material of this invention is used as a prosthetic appliance of bone.
Figure 14a, Figure 14b are perspective views showing further the other embodiments of this invention.

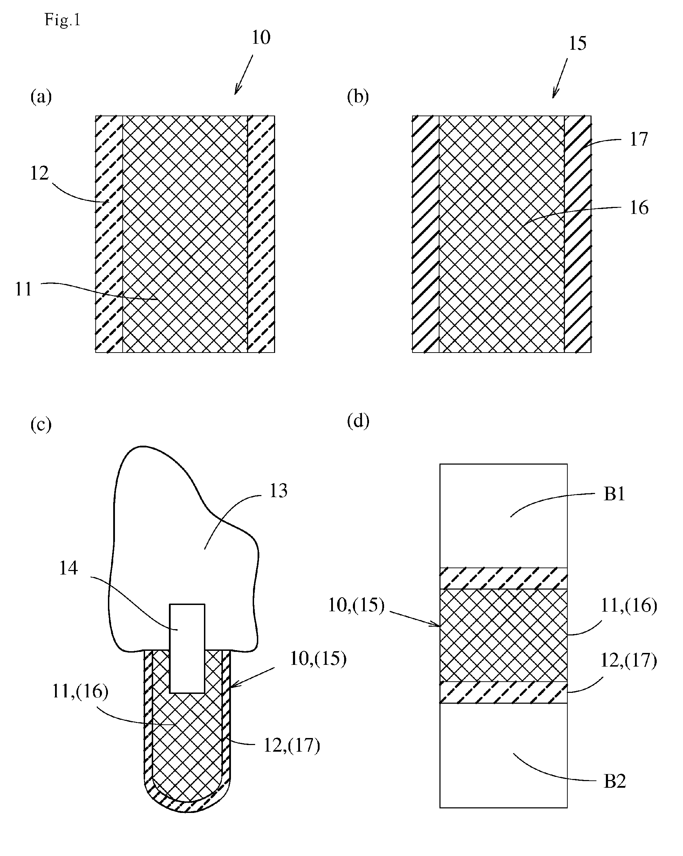

A medical material 10 shown in Figure 1a can be used for an artificial tooth root (see Figure 1b) or an artificial bone (see Figure 1c) etc. It comprises a support 11 composed of titanium web and an outer layer 12 composed of bioabsorbable calcium phosphate compound formed in the periphery thereof.

The support 11 is formed of a metal nonwoven fabric which is made by twisting or intertwining titanium or titanium alloy metal fibers. The metal fiber whose diameter is 100µm or less is used. But the fiber may have quadrilateral shape whose one side is 100µm or less. Further, it may be polygonal shape whose one side is 100µm or less. The porosity of the support 11 is formed so as to be 50-95%, particularly to be 80-90%. The reason is because many kinds of cell preferably grow and positively adhere to or couples with the support 11 having such a geographic space structure composed of fine fibers. Thereby, the period for cells to spread around the support and to form physical combination with the support is shortened.

When this medical material is used as an artificial tooth root (see Figure 1), the diameter of the support 11 is preferable to be 2-10 mm, particularly 3-6 mm, and it is preferable to be 10-50%, particularly 20-30% to the overall diameter of the medical material 10. Thereby, the strength as the artificial tooth root is given, and shear etc. between the replaced bone tissues does not occur. Therefore, the medical material is equip with sufficient strength as the artificial tooth root, even when after the outer layer 12 composed of bioabsorbable calcium phosphate compound is replaced with bone tissues.
Moreover, when this medical material is used as an artificial bone (Figure 1d), though being different depending on the portion where the bone is used, the diameter of the support 11 is preferable to be 5-40 mm, particularly 10-30 mm, and it is also preferable to be 10-50%, particularly 20-30% to the overall diameter of the medical material 10. Thereby, the strength as the artificial tooth root is given, and shear etc. between the replaced bone tissues will be prevented, making it possible to keep sufficient strength as the artificial tooth root, even when the outer layer 12 composed of bioabsorbable calcium compound is replaced with bone tissues.

On the surface of metal fiber of the support 11, bioactive substance or bioactive auxiliary agent etc. may be coated. As such bioactive substance or bioactive auxiliary agent, cell growth promoting factor, cytokine, antibiotics, cell growth controlling factor, enzyme, protein, polysaccharide, phospholipid, lipoprotein, and mucopolysaccharide can be cited. By these, the induction of cell can be promoted.

The bioabsorbable outer layer 12 is composed of bioabsorbable calcium phosphate compound such as α-tricalcium phosphate, β-tricalcium phosphate, tetracalcium phosphate, apatite hydroxide deposited from a solvent etc. Particularly, when the apatite hydroxide deposited from the solvent etc. is used as the outer layer 12, the thickness of the outer layer 12 can be controlled by the immersion time to the solvent. The outer layer 12 has pores of 50-500 µm, particularly, 200-400µm, the porosity of which is 50-95%, particularly, 50-80%. By providing the outer layer 12 as thus, cells will be favorably induced into the space of the outer layer 12, and couple with the outer layer 12 chemically and physically.

When the medical material is used as an artificial tooth root, the outer layer 12 is preferable have thickness of 0.01-1mm, particularly 0.05-0.5 mm, and its thickness is preferable to be 0.1-10%, particularly 0.2-5%, to the overall diameter of the medical material 10. And, when the medical material is used as an artificial bone, the outer layer 12 is preferable to have thickness of 0.01-2mm, particularly 0.05-1 mm, and its thickness is preferable to be 0.1-10%, particularly 0.2-5% to the overall diameter of the medical material 10. Thereby, the induced cells coupled firmly to the outer layer 12, and the shear between the support 11 and the outer layer 12 will be prevented even though the external force is applied. Therefore, the binding strength between the medical material and the living body, required for medical treatment can be obtained. Further, though being different according to the kind of the bioabsorbable calcium phosphate compound, cells will sufficiently induced into and bond with the support 11 before the ceramic layer is completely replaced by the induced cells, and the binding strength between the support 11 and the cells is obtained.

Moreover, a bioactive substance or a bioactive auxiliary agent to activate the biological cells may be charged in the outer layer 12.

Such medical material 10 is, preferable to be formed so that the overall diameter is 2-10mm, particularly 3-6 mm, when used as an artificial tooth root; and preferable to be formed so that the overall diameter is 5-40 mm, particularly, 10-30mm, when used for an artificial bone.
The medical material formed as thus, is inserted into the living body so that the outer layer 12 contacts the biological cells, therefore the cells of the living body will be induced into the medical material. In other words, the induced cells couples with the outer layer 12 at the beginning, then expand into the support 11 through the outer layer 12, and couples with the support 11.

When the medical material 10 is used as an artificial tooth root as shown in Figure 1c, the outer layer 12 is provided in the periphery excluding the upper surface of the support 11, and it is inserted into a jaw bone (alveolar bone). Then, bone cells expand into the medical material 10. After the medical material 10 and the cells bonds sufficiently, an abutment 14 to which the artificial tooth root 13 is attached in the upper surface of the support is fitted in.
In the medical material 10 used as an artificial bone, as shown in Figure 1d, the support 11 is formed to be in the outer shape of a bone or to be in a tube shape. The outer layer 12 is formed in its upper and lower surfaces of the support. And, this medical material (artificial bone) 10 is inserted between bones B1, B2 to be joined so that the outer layer 12 contacts the bone B1, B2. Thereby, the bone cells are induced from the outer layer 12, couples with the outer layer 12, expand inside, and couples with the support 11. Hence, early healing is possible with the firm bonding of the cells and the medical material couples. Moreover, when it is used as an artificial bone, the ablation of the treatment portion after treatment can be done easily by taking out the support 11, since the outer layer 12 will be replaced by bone cells after treatment.

This medical material 10 is manufactured as follows. At the beginning, a metal nonwoven fabric made by intertwining titanium alloy metal fibers whose average diameter is 100 µm or less, is prepared. Then, this is inserted into a mold previously prepared, and the support 11 is fabricated by compressing it so as to make the porosity 50-95%, particularly 80-90%. Here, the fabricated support 11 may be inserted into a mold jig made of ceramics and may be sintered at a temperature of 500-1500 degrees lower than the melting temperature (1668 degrees) of titanium.
Next, bioabsorbable ceramics is covered on the periphery of the support 11. As the method for covering, the solution technique is preferable. Using the solution technique, the thickness of the covering may be controlled arbitrary.

The medical material 15 of Figure 1b comprises a support 16 composed of titanium web and an outer layer 17 composed of non-bioabsorbable calcium phosphate compound formed in the periphery thereof. Stated differently, the material of the outer layer of the medical material 10 of Figure 1a is changed to non-bioabsorbable material.

This support 16 is formed from a metal nonwoven fabric which is made by twisting titanium or titanium alloy metal fibers having the diameter of 100 µm or less and its porosity is made to be 50-95%, preferably 80-90%, same as the support 11 of the first embodiment.

The support 16 can be used as an artificial tooth root or as an artificial bone same as the support 11 of Figure 1a. And, its size also is substantially same as the support 11 of Figure 1a.

The non-bioabsorbable outer layer 17 is composed of non-bioabsorbable calcium phosphate compound such as apatite hydroxide. And, the pores of the outer layer 17 is 50-500 µm in diameter, particularly, 200-400 µm in diameter, and its porosity is formed to be 50-80%, particularly, 50-60%. By providing such outer layer 17, the induction of cells is promoted and the period to form the binding of the outer layer 17 and the cells will be shortened.

The thickness of such outer layer 17 is preferable to be 0.01-1 mm, particularly 0.05- 0.5 mm and it is preferable to be 0.1-10%, particularly 0.2-5% to the overall diameter of the medical material 15 when used as an artificial tooth root. And, the thickness is preferable to be 0.01-2 mm, particularly 0.05- 0.1 mm and it is preferable to be 0.1-10%, particularly 0.2-5% to the overall diameter of the medical material 10 when used as an artificial bone.
Since being composed as thus, the outer layer 17 couples with the bone without being absorbed by a living body. Hence, the induction of cells will be slow compared with the medical material 10 of Figure 1, but the strength after surgical treatment increases. However, even if it is bio-nonabsorbable ceramics, the outer layer will be absorbed and replaced with the passage of time, year by year. Further, in the medical material 15 equipped with the outer layer 17, it prevents the shear between the support 16 and the outer layer 17, and the binding strength between the medical material and the living body is secured.

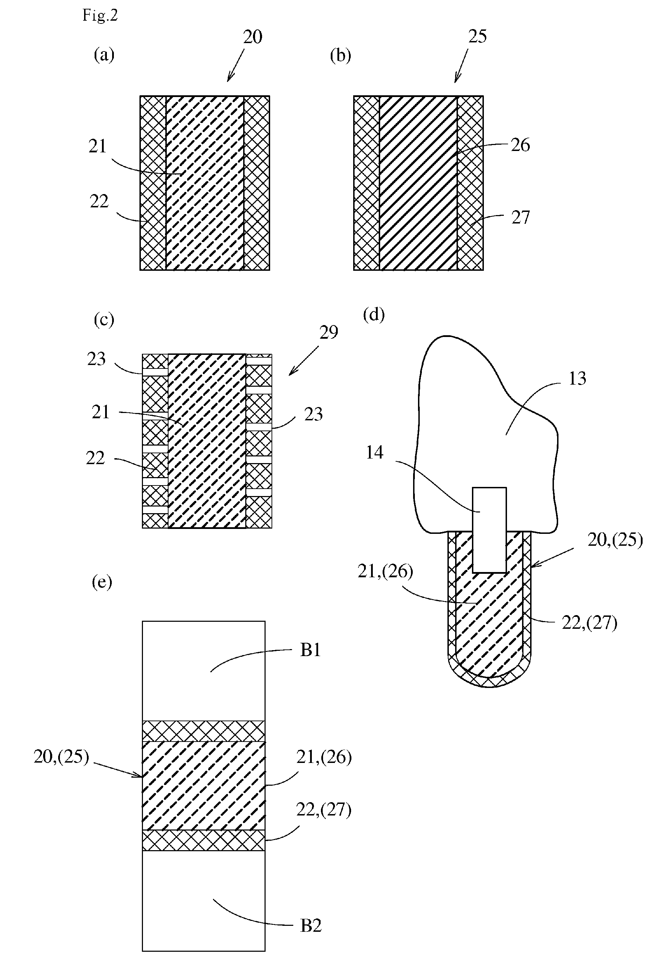

The medical material 20 shown in Figure 2a has support and the outer layer like medical material of Figure 1a, although the material of the support and the outer layer are reversed. In other words, the medical material 20 comprises a support 21 composed of bioabsorbable calcium phosphate compound and an outer layer 22 composed of titanium web.

This bioabsorbable support 21 is composed of bioabsorbable calcium phosphate such as α -tricalcium phosphate, β -tricalcium phosphate, tetracalcium phosphate. The support 21 has pores having the diameter of 50-500 µm, particularly 200-400 µm, and support 21 is formed so that the porosity is 50-95%, particularly 50-80%. When the medical material 20 is as tooth root (see Figure 2d), the diameter of the support is preferably to be 2-10mm, particularly 3-6mm, and it is preferable to be 10-50 %, particularly 20-30 % to the overall diameter of the medical material 20. And, though it is different according to the portion where the bone is used, when the medical material 20 is used as an artificial bone, the diameter of the support 21 is preferable to be 5-40mm, particularly 10-30 mm, and the diameter of the support 21 is preferable to be 10-50 %, particularly 20-30 % to the overall diameter of the medical material 20.

This outer layer 22 is composed of a metal nonwoven fabric which is made by intertwining titanium or titanium alloy metal fibers of which the diameter is 100 µm or less. The porosity of the support is 5- 95 %, preferably 80-90%. The thickness of the outer layer 22 is 1-10 mm, particularly 1-6mm, most preferably 1-2 mm when the medical material 20 is used as an artificial tooth root (see Figure 2d). And, its thickness is preferable to be 10-50 % particularly 10-30 % to the overall diameter of the medical material 20.
And, when the medical material is used as an artificial bone (see Figure 2e), the thickness is preferable to be 1-10 mm, particularly 1-6 mm, and is 5-50 % particularly 5-30 % to the overall diameter of the medical material 20.

Since it is composed as thus, cells expand into the outer layer 22 at the beginning and couples with the outer layer 22. Then, the cells reached the support 21 through the outer layer 22 and couples with the support 21. And, since the support 21 will be replaced with bone cells, only the cylindrical outer layer 22 composed of titanium web remains. This remaining works as a strength reinforcing member. Hence, the outer layer 22 may be removed or not after treatment according to the therapeutic situation.
Such medical material 20 is preferably to be use as the artificial tooth root or the artificial bone for an aged person etc., who has trouble inducing cells, because even if the density of bone cells which are induced into the support and replaced with the support, is low, the whole will be reinforced by outer layer 22.

The medical material 25 shown in Figure 2b is equipped with a support 26 composed of non-bioabsorbable calcium phosphate compound and an outer layer 27 composed of titanium web formed in the periphery thereof.

The non-bioabsorbable support 26 is composed of non-bioabsorbable calcium phosphate compound such as apatite hydroxide. The support 26 has pores of the diameter to be 50-500 µm, particularly to be 200-400 µm, and is formed so that the porosity is 50-80 %, particularly 50-60%. When medical material 25 is used as an artificial tooth root (see Figure 2d), the diameter of the support 26 is preferably to be 2-10mm, particularly to be 3-6mm, and the diameter of the support 26 is preferable to be 10-50 %, particularly 20-30 % to the overall diameter of the medical material 25. And, when the medical material 25 is used as an artificial bone (see Figure 2e), though being different according to the portion where the bone is used, the diameter of the support 26 is preferable to be 5-40mm, particularly 10-30 mm, and it is preferably to be 10-50 %, particularly 20-30 % to the overall diameter of the medical material 25.

This outer layer 27 is formed from a metal nonwoven fabric made by intertwining titanium or titanium alloy metal fibers of which the diameter is 100 µm or less, and the porosity is 50-95 %, preferably 80-90%. The thickness of such outer layer 27 is preferable to be 0.5-5 mm, particularly 0.5-2 mm, and 10-50%, particularly 20-30% to the overall diameter of the medical material 25, when used as an artificial tooth root. And, the thickness is preferable to be 5-40 mm, particularly 10- 30 mm, and is to be 10-50%, particularly 20-30% to the overall diameter of the medical material 25 when used as an artificial bone (see Figure 2e).

Since being composed as thus, cells expand into the outer layer 27 and couples with the outer layer 27, then couples with the support 26 same as the medical material 20.
Medical material 25 is preferably to be used as the artificial tooth root or the artificial bone for an aged person etc. who has difficulty inducing cells, because even if the density of bone cells induced into the support is low, the whole is reinforced by outer layer 22. Moreover, since the support will not be replaced with the induced low density bone, the whole is further strengthen.

Moreover, as the medical material 29 shown in Figure 2c, a plurality of pathways 23 to communicate with the outside and the support 21 may be provided in the outer layer 22 of Figure 2a. This is to induce cells first into the support 21 through the pathways 23 and to form the coupling of the cells and the support 21 first. After that, cells are to expand into the outer layer 22 from the pathways 23 and the periphery of the support 21, and cells are to form the coupling with the outer layer 22. Since the binding or coupling of the support 21 and the cells can be obtained in first as above, the binding of the medical material and the living body can be obtained in a comparatively early stage, after the medical material 20 is implanted into a living body. The pathway 23 may be provided in the outer layer 27 of the medical material 25 of Figure 2b.

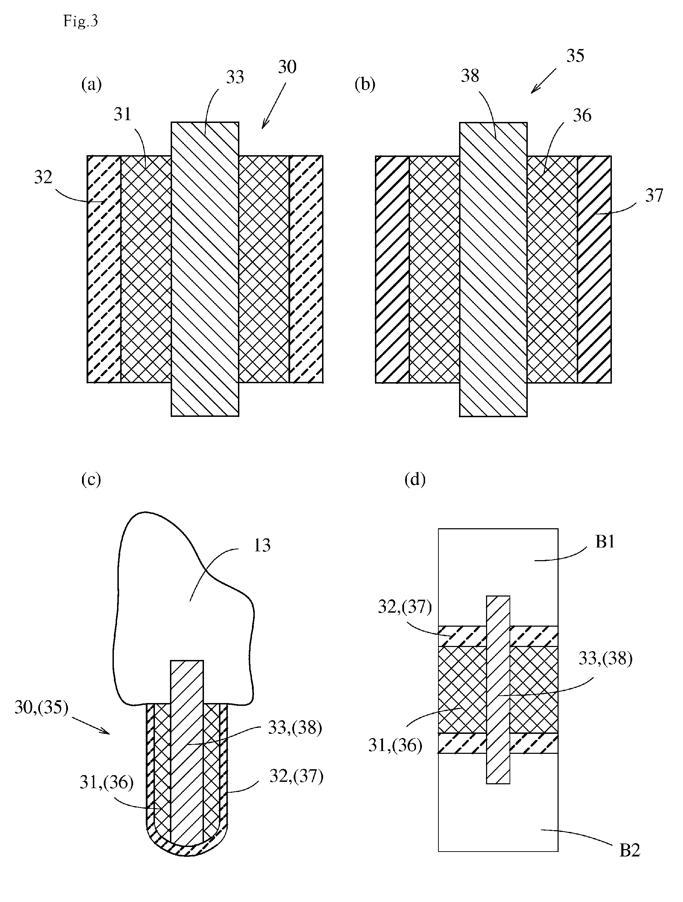

The medical material 30 of Figure 3a comprises a cylindrical inner layer 31 composed of titanium web, an outer layer 32 composed of bioabsorable calcium phosphate compound formed in the periphery of the inner layer, and a rod 33 inserted into the inner layer.

The inner layer 31 of the medical material 30 is formed from a metal nonwoven fabric which is made by intertwining titanium or titanium alloy metal fibers of which the diameter is 100 µm or less, and its porosity is 50-95 %, preferably 80-90%. The thickness of such inner layer 31 is preferable to be, 2-10 mm, particularly 3-6 mm, and it is preferably to be 10-50%, particularly 20-30 % to the overall diameter of the medical material 30, when the medical material 30 is used as an artificial tooth root (see Figure 3c). And, the thickness is preferable to be 5-40 mm, particularly 10-30 mm, and it is preferably to be 10-50 %, particularly 20-30 % to the overall diameter of the medical material 30 when medical material 30 is used as an artificial bone (see Figure 3d).

This bioabosorbable outer layer 32 is composed of bioabsorbable calcium phosphate system material such as α-tricalcium phosphate, β-tricalcium phosphate, tetracalcium phosphate. The pores of the diameter are preferably to be 50-500 µm, particularly 200-400 µm, and its porosity is preferably to be 50-80 %, particularly 50-60%. The thickness of the outer layer 32 is 1-3mm, particularly 1-2 mm, and the thickness of outer layer 32 is preferable to be 2-20 %, particularly 5-10 % to the overall diameter of the medical material 30, when the medical material 30 used as an artificial tooth root (see Figure 3c). And, though being different according to the portion where the bone is used, the thickness of the outer layer 32 is preferable to be 1-30mm, particularly 1-20 mm, and is preferably to be 2-20 %, particularly 5-10 % to the overall diameter of the medical material 30, when the medical material 30 is used as an artificial bone (see Figure 3d).

The rod 33 is a rod made of stainless steel or titanium and is for strengthening the medical material. Here, the use of titanium rod allows undergoing MRI examination while in medical treatment, which is preferable. Moreover, the rod 33 keeps sufficient strength, even when bone tissues are not induced sufficiently or even when the bone density is low. Therefore, the medical material 30 is used also for a person who is comparatively slow or difficult to induce cells such as an aged person, or for such a portion of osteoporosis, osteomalacia etc. where the induction of cells is difficult. The diameter of such a rod is preferable to be 2-20 mm, particularly 3- 10 mm, and it is preferably to be 5-50 %, particularly 10-20% to the overall diameter of the medical material 30.
Since the rod 33 acts as an axis, the strength of the medical material 30 can be kept, which is preferable.

In the medical material 30, cells are induced by being implanted in a living body. The induced cells couple chemically with the outer layer 32 at the beginning, and then couple physically. Further cells reach the inner layer 31 while expanding into the outer layer 32. And, they couple with the inner layer 31 by expanding into the inner layer 32.
This medical material 30 can also be used as an artificial tooth root (see Figure 3c), and an artificial bone (see Figure 3d). When this medical material is used as an artificial tooth root, the rod 33 can be used as an abutment for attaching the rod to an artificial tooth 34.
Moreover, the medical material 30 may comprises the cylindrical outer layer 32(top), the cylindrical inner layer 31, and the cylindrical outer layer 32(bottom) formed in sequence from the top, and the rod 33 formed so as to penetrate them and to protrude above and below. This medical material 30 may be used as an artificial bone, so that the protruding rod 33 stabs in a bone B1 and a bone B2 to be joined, allowing the medical treatment while the strength of the treated area is kept as it is.

A medical material 35 comprises a cylindrical inner layer 36 composed of titanium web, an outer layer 37 composed of bioabsorbable calcium phosphate compound formed in the periphery of the inner layer , and a rod 38 inserted into the inner layer.

The inner layer 36 of the medical material 35 is formed from a metal nonwoven fabric which is made by intertwining titanium or titanium alloy metal fibers of which the diameter is 100 µm or less, and its porosity is 50-95 %, preferably 80-90%. When the medical material 35 is used as an artificial tooth root (see Figure 3c), the diameter of such inner layer 36 is preferable to be, for example, 2-10 mm, particularly 3-6 mm, and it is preferably to be 10-50%, particularly 20-30 % to the overall diameter of the medical material 35. And, when the medical material 35 is used as an artificial bone (see Figure 3d), the diameter of the inner layer 36 is preferable to be 5-40 mm, particularly 10-30 mm, and it is preferably to be 10-50 %, particularly 20-30 % to the overall diameter of the medical material 35.

The outer layer 37 of the medical material 35 is composed of non-bioabsorbable calcium phosphate compound material such as apatite hydroxide. The outer layer 37 may have pores whose diameter is 50-500 µ m, particularly 200-400 µm, and its porosity is preferably to be 50-80%, particularly 50-60%. When the medical material 35 is used as an artificial tooth root (see Figure 3c), the thickness of the outer layer 37 is preferably to be 1-3 mm, particularly 1-2 mm, and the diameter of the outer layer 37 is preferable to be 2-20 %, particularly 5-10 % to the overall diameter of the medical material 35. And, when the medical material 35 is used as an artificial bone (see Figure 3d), though being different according to the portion where the bone is used, the diameter of the outer layer 37 is preferable to be 5-40mm, particularly 10-30 mm, and it is preferably to be 10-50 %, particularly 20-30 % to the overall diameter of the medical material 35.

The rod 38 is for strengthening the medical material 35, and is substantially same as the rod 33 of Figure 3a.
Since this medical material 35 is also equipped with the rod 38 it can keep the strength while in medical treatment same as medical material 30.

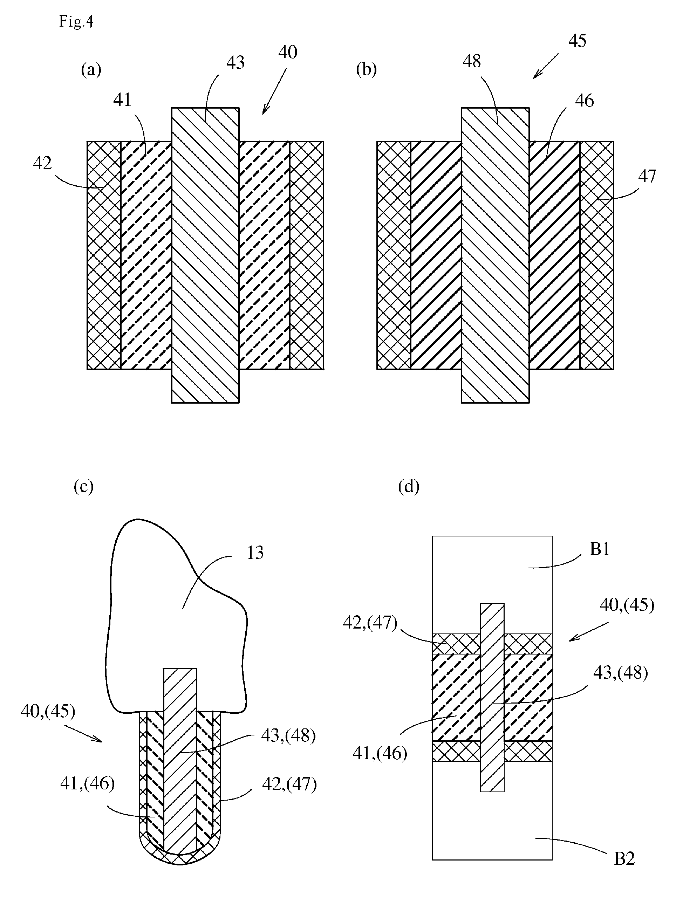

A medical material 40 of Figure 4 comprises an inner layer 41 composed of bioabsorbable calcium phosphate compound and an outer layer 42 composed of titanium web formed in the periphery of the inner layer, and a rod 43 inserted into the inner layer.

This bioabosorbable inner layer 41 is composed of bioabsorbable calcium phosphate compound such as α-tricalcium phosphate, β-tricalcium phosphate, tetracalcium phosphate. It has pores of the diameter 50-500 µm, particularly 200-400 µm, and its porosity is 50-80 %, particularly 50-60%. The thickness of such inner layer 41 is preferable to be 2-10 mm, particularly 3-6 mm (in the case of artificial tooth root), it is preferably to be 5-40 mm, particularly 10-30mm (in the case of artificial bone), and it is preferably to be 10-50 %, particularly 20-30 % (in the case of artificial tooth root, artificial bone) to the overall diameter of the medical material 40.

This outer layer 42 of the medical material 40 is formed from a metal nonwoven fabric which is made by intertwining titanium or titanium alloy metal fibers, whose porosity of the support is 50- 95 %, preferably 80-90%. The thickness of the outer layer 42 is preferable to be 1-3 mm, particularly 1-2mm, and it is preferably to be 2-20%, particularly 5-10 % to the overall diameter of the medical material 40.

The rod 43 of the medical material 40 is substantially same as the rod 33 of Figure 3a.
This medical material 40 can also be used as an artificial tooth root and as an artificial bone as shown in Figure 4c and Figure 4d.
Moreover, this medical material is provided with an outer layer composed of titanium web in the periphery of the inner layer composed of ceramics, same as the medical material 20. Therefore, high strength artificial tooth roots, artificial bones can be obtained, even when an aged person who is difficult to induce cells is the target. However, induction of cells becomes slow compared with the medical material 30.

A medical material 45 of Figure 4b comprises a cylindrical inner layer 46 composed of non-bioabsorbable calcium phosphate material and an outer layer 47 composed of titanium web formed in the periphery of the inner layer , and a rod 48 inserted into the inner layer,

This inner layer 46 is composed of non-bioabsorbable calcium phosphate compound of apatite hydroxide. It has pores having the diameter of 50-500 µm, particularly 200-400 µm, and its porosity is 50-80 %, particularly 50-60%. The thickness of the inner layer 41 is preferable to be 2-10 mm, particularly 3-6 mm (in the case of artificial tooth root), or it is preferably to be 5-40 mm, particularly 10-30mm (in the case of artificial bone). And it is preferable to be 2-20 %, particularly 5-10 % (in the case of artificial tooth root) to the overall diameter of the medical material 40, or it is preferably to be 2-20%, particularly 5-10 % (in the case of artificial bone) to the overall diameter of the medical material 40.
This has the same reinforcing action like the medical material 40. Moreover, since the inner layer is not replaced by bone cells, it has higher strength than the medical material 40.

The outer layer 47 of the medical material 45 is formed from a metal nonwoven fabric which is made by intertwining titanium or titanium alloy metal fibers of the diameter 100 µm or less. The porosity of the outer layer 47 is 50-95 %, preferably 80-90%. The thickness of the outer layer 47 is preferable to be 1-3 mm, particularly 1-2mm (in the case of artificial tooth root, Figure 4c) and it is preferably to be 1-3mm, particularly 1-2mm (in the case of artificial bone, Figure 4d). And it is preferable to be 2-20%, particularly 5-10 % to the overall diameter of the medical material 40 (in the case of artificial tooth root, Figure 4c), or it is preferably to be 2-20%, particularly 5-10 % (in the case of artificial bone, Figure 4d) to the overall diameter of the medical material 40.

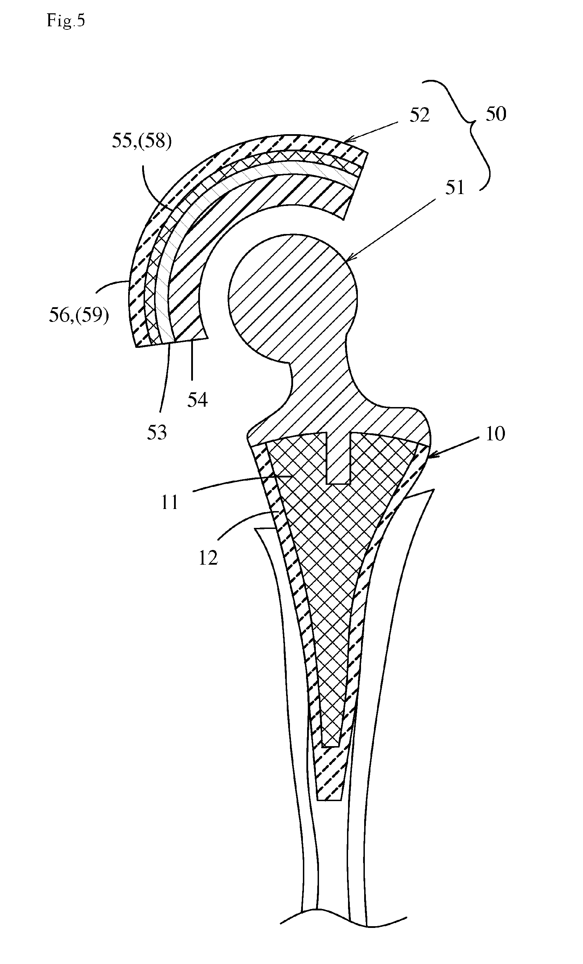

The medical material of Figure 5a is used for an artificial joint. This artificial joint 50 comprises an artificial osteomere and a tray 52. As the artificial osteomere 51, titanium or titanium alloy is used. This artificial osteomere 51 is provided on the upper end of the medical material 10 of Figure 1 used as an artificial bone attached to a bone B as shown in the figure.
The tray 52 of the artificial joint 50 comprises a fan-shaped metal substrate 53, a synthetic resin layer 54 provided on the inner surface of the metal substrate, a metal fiber layer 55 composed of titanium web provided on the outer surface of the metal substrate, and a ceramics layer 56 provided on the outer surface of the metal fiber layer.

As the metal substrate 53, stainless steel or titanium of high bio-compatibility material may be used. Particularly, titanium is preferable because it does not give impact on MRI examination etc.
As the synthetic resin layer 54, synthetic resin such as polymer polyethylene is preferable considering the slip and the durability with the artificial osteomere 61. Its thickness is preferable to be 2-20 mm, particularly 5-10 mm.

As the metal fiber layer 55, a metal nonwoven fabric of titanium or titanium alloy metal fiber which is intertwined and which have the diameter of 100 µm or less, and the porosity of 50-95%, preferably 80-90% is used. The thickness is preferable to be 1-10 mm, particularly 3-5 mm.

Bioabosorbable calcium phosphate compound such as α-tricalcium phosphate, β-tricalcium phosphate, tetracalcium phosphate may be used as the ceramic layer 56. In this case the diameter of the pores is preferably to be 50-500 µm, particularly 300-400 µm, and the porosity is preferably to be 50-80%, particularly 50-60%. The thickness is preferable to be 1-10 mm, particularly 2-5 mm.
Moreover, non-bioabsorbable calcium phosphate such as apatite hydroxide may be used as the ceramic layer. In this case the diameter of the pore is preferably to be 50-500 µm, particularly 300-400 µm, and the porosity is preferably to be 50-80%, particularly 50-60%. Its thickness is preferable to be 1-10 mm, particularly 2-5 mm.
And, when a bioabsorbable compound is used as the ceramics layer 56, since it will be replaced with bone, final biocompatibility with a living body is good. Moreover, when a non-bioabsorbable compound is used as the ceramics layer 56, the strength can be obtained regardless of the induction circumstance of living body.

By implanting the tray 52 of artificial joint composed as thus, the induced bone cells couple with ceramics layer 56, then, induced to the metal layer 55 through the ceramics layer, and couples with metal fiber layer 55.

And, the metal fiber layer 55 of Figure 5 and the ceramics layer 56 may be arranged reversely. Stated differently, the other configuration of the tray 52 of the artificial joint comprises a fan-shaped metal substrate 53, a synthetic resin layer 54 provided on the inner surface of the metal substrate, a ceramics layer 58 provided on the outer surface of the metal substrate, and a metal fiber layer 59 composed of titanium web provided on the outer surface of the ceramics layer.

As the ceramics layer 58, bioabosorbable calcium phosphate compound such as α-tricalcium phosphate, β-tricalcium phosphate, tetracalcium phosphate may be used. In this case the diameter of pores is preferably to be 50-500 µm, particularly 300-400 µm, and the porosity is preferably to be 50-80%, particularly 50-60%. Its thickness is preferable to be 1-10 mm, particularly 2-5 mm.
Moreover, non-bioabsorbable calcium phosphate compound such as apatite hydroxide may be used as the ceramic layer 58. In this case, the diameter of pores is preferably to be 50-500 µm, particularly 300-400 µm, and the porosity is preferably to be 20-60%, particularly 30-50%. Its thickness is preferable to be 1-10 mm, particularly 2-5 mm.

As the metal fiber layer 59, a metal nonwoven fabric which is made by intertwining titanium or titanium alloy metal fibers having diameter 100 µ m or less, of which the porosity is 50-95%, preferably 80-90% is used. Its thickness is preferable to be 1-5 mm, particularly 1-3 mm.

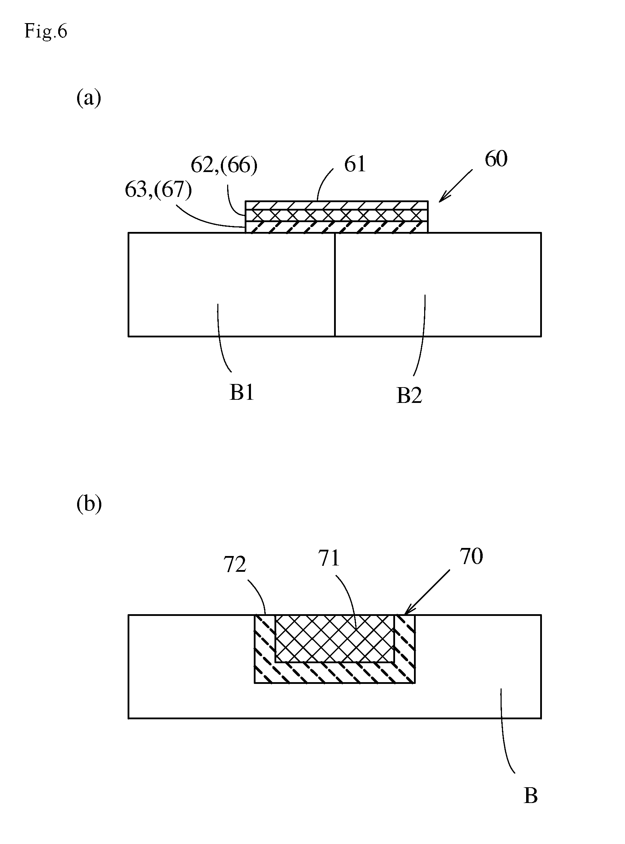

The medical material shown in Figure 6a is that which is used for a bone plate 60. This bone plate 60 comprises a metal substrate 61, a metal fiber layer 62 composed of titanium web provided on its inner surface, and a ceramics layer 63 composed of calcium phosphate compound.

As the metal fiber layer 62, a metal nonwoven fabric which is made by intertwining titanium or titanium alloy metal fibers having diameter 100 µm or less, of which the porosity is 50-95%, preferably 80-90% is used. Its thickness is preferable to be 1-10 mm, particularly 1-5 mm.

As the ceramics layer 63, bioabosorbable calcium phosphate compound such as β-tricalcium phosphate may be used. In this case the diameter of pore is preferably to be 50-500 µm, particularly 300-400 µm, and the porosity is preferably to be 20-60%, particularly 30-50%. Its thickness is preferable to be 1-10 mm, particularly 2-5 mm. Moreover, non-bioabsorbable calcium phosphate compound such as apatite hydroxide may be used as the ceramic layer 63. In this case the diameter of pores is preferably 50-500 µm, particularly 300-400 µm, and the porosity is preferably to be 20-60%, particularly 30-50%. Its thickness is preferable to be 1-10 mm, particularly 2-5 mm.

By joining the separated bone B1 and B2 with such bone plate 60 using a screw etc, bone cells are induced into the bone plate 60, couples with ceramics layer 63, further couples with the metal fabric layer 62. Resultantly firmly joins the bone B1 and the bone B2.

The arrangement of the metal fiber layer 62 and the ceramics layer 63 may be reversed. Stated differently, the other configuration of the bone plate 60 comprises a metal substrate 61, a ceramics layer 66 composed of calcium phosphate compound provided on its inner surface, and a metal fiber layer 67 composed of titanium web. The metal substrate 61 is substantially same as that of Figure 6a.

As the ceramics layer 66, bioabosorbable calcium phosphate compound such as α-tricalcium phosphate, β-tricalcium phosphate, tetracalcium phosphate may be used. In this case the diameter of pores is preferably to be 50-500 µm, particularly 300-400 µm, and the porosity is preferably to be 70-95%, particularly 80-90%. Its thickness is preferable to be 1-10 mm, particularly 2-5 mm.
And, non-bioabsorbable calcium phosphate compound such as apatite hydroxide may be used as the ceramic layer 66. In this case the diameter of pores is preferably to be 50-500 µm, particularly 300-400 µm, and the porosity is preferably to be 30-50%, particularly 40-50%. Its thickness is preferable to be 1-10 mm, particularly 2-5 mm.

As the metal fiber layer 67, a metal nonwoven metal fabric which is made by intertwining titanium or titanium alloy metal fibers of the diameter 100 µm or less, of which the porosity is 50-95%, preferably 80-90% is used. Its thickness is preferable to be 1-10 mm, particularly 3-5 mm.

The medical material 70 of Figure 6b is used for a prosthetic appliance of bone, and comprises a substrate 71 and a covering layer 72 composed of calcium phosphate compound provided so as to cover the substrate.

The substrate 71 is formed from a metal nonwoven fabric which is made by intertwining titanium or titanium alloy metal fibers of the diameter 100 µm or less, of which the porosity is 50-95%, preferably 80-90% is used. The size of the substrate 71 is adjusted according to the size of defect or prosthetic portion of bone.
As the covering layer 72, the diameter of pores is preferably to be 50-500 µm, particularly 300-400 µm, and the porosity is preferably to be 30-60%, particularly 40-50%, when bioabosorbable calcium phosphate compound such as α-tricalcium phosphate, β-tricalcium phosphate, tetracalcium phosphate are used. Further, its thickness is formed to be 1-10 mm, particularly 2-5 mm.
As the covering layer 72, the diameter of pores is preferably to be 50-500 µm, particularly 300-400 µm, and the porosity is preferably to be 30-60%, particularly 40-50%, when non-bioabsorbable calcium phosphate compound such as apatite hydroxide is used. Further, its thickness is preferable to be 1-10 mm, particularly 2-5 mm.

In this prosthetic appliance 70 of bone, when it is inserted into the defect of bone, the bone cells induced from the prosthetic appliance, couple with the covering layer 72. Then, the bone cells expand and reach the substrate 71 through the covering layer 72 to couple with the substrate 71.

In this prosthetic appliance 70 of bone also, the arrangement of the substrate 71 and the covering layer 72 may be reversed.
In this case, as the substrate 71, the diameter of pores is preferably to be 50-500 µm, particularly 300-400 µm, and the porosity is preferably to be 30-60%, particularly 40-50%, when bioabsorbable calcium phosphate compound such as α-tricalcium phosphate, β-tricalcium phosphate, tetracalcium phosphate. Further, the thickness can be adjusted according to the size of the defect and the size of the prosthetic portion.
And, as the substrate 71, the diameter of pores is preferably to be 50-500 µm, particularly 300-400 µm, and the porosity is preferably to be 30-60%, particularly 40-50% when non-bioabsorbable calcium phosphate compound such as apatite hydroxide is used. The size of the base material 71 is adjusted according to the size of the defect and the size of the prosthetic portion.

And, in this case, as the covering layer 72, a metal nonwoven fabric which is made by intertwining titanium or titanium alloy metal fibers of the diameter 100 µm or less, of which the porosity is 50-95%, preferably 80-90% is used. Its thickness is preferable to be 1-10 mm, particularly 2-5 mm.

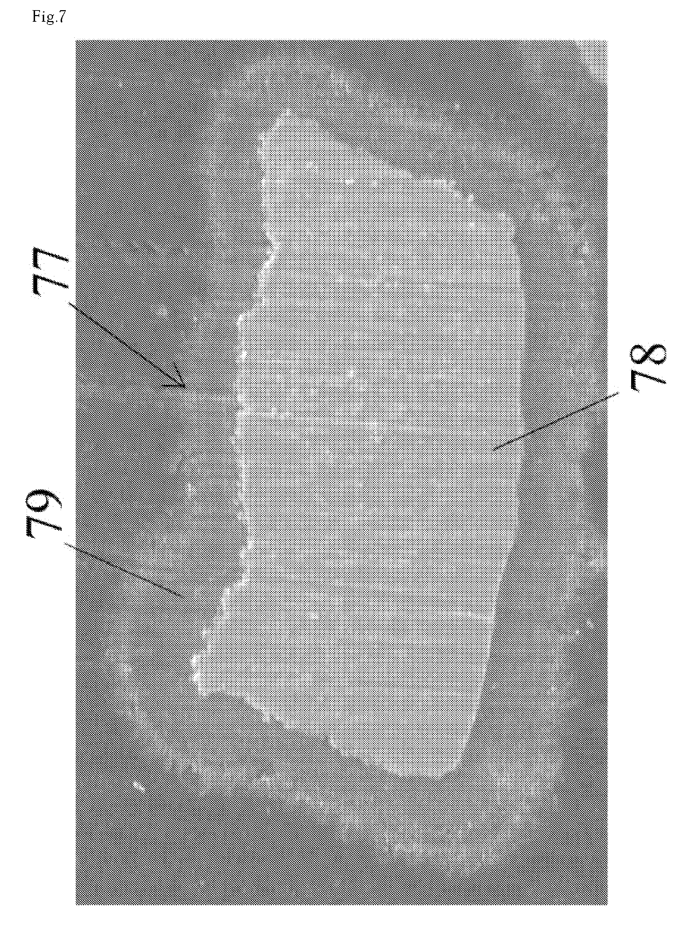

The medical material 77 shown in Figure 7 comprises a substrate 78 and a coat layer 79 provided in its exterior. Here, the substrate 78 is titanium metal fibers of about rectangular cross section, and the coat layer 79 is apatite hydroxide which is non-bioabsorbable ceramics. According to this electron microscope photograph, it is found that the coat layer 79 is uniformly provided around the substrate 78.

As the metal fiber of medical materials disclosed so far, those which have circular cross section of the diameter 100 µm or less have been mainly disclosed. But they may be those which have rectangular cross section whose one side is 100 µm or less. Moreover, they may be polygon whose one side is 100 µm or less.

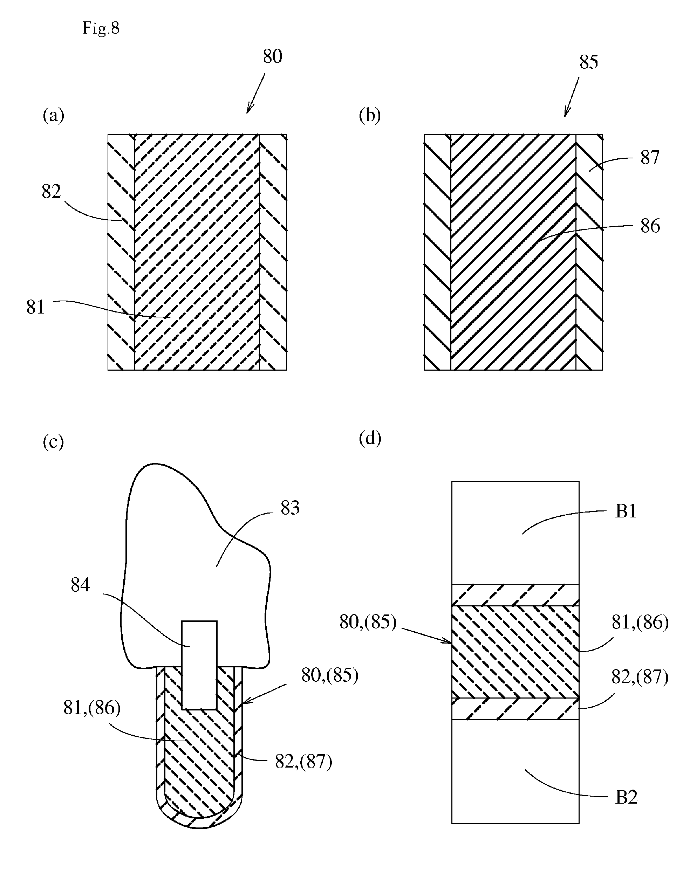

The medical material 80 shown in Figure 8a is that which can be used for an artificial tooth root (see Figure 8c) or an artificial bone (see Figure 8d). It comprises a support 81 composed of bioabsorbable ceramics whose porosity is 0.1-10 %, particularly 1-5 %, and an outer layer 82 composed of bioabsorbable ceramics whose porosity is 11-80 % (high porosity), particularly 50-70 % provided in the periphery thereof. The difference of the porosity of the support 81 and the outer layer 82 is 10-80 points.

Since the support 81 of such medical material is composed of bioabsorbable ceramics having the porosity of 0.1-10 % (low), extracellular matrix components such as collagen, fibronectin, alubmin can be loaded inside of the support. The loading of the extracellular matrix components promotes cell proliferation, cell differentiation in the support. Hence, cells can be induced inside of the support to exert anchor effect in the fixation with a living body. Moreover, the strength as a whole of the medical material 80 can be obtained. After the medical material is implanted into a living body, the medical material is fixed to the living body until the cells are induced. In other words, the introduction of extracellular matrix components into the support 81 makes it easy for the cells to enter into the layer 82, and can heighten the strength as a whole, comparatively shortening the induction period to the whole of the support.

At the same time, the outer layer 82 is composed of bioabsorbable ceramics whose porosity is 11-80 % (high), particularly 50-70 %, where the difference to the porosity of the support is 10-80 points. Further, the diameter of the pore is 50- 500 µm, particularly 300-400 µm. This porosity of the outer layer 82 is a geometric structure in which cells most preferably infiltrate or invades. And, since the outer layer 82 is formed so that the porosity is 10-80 points larger than that of the support, it has a role to induce cells infiltrated preferably into the outer layer 82 into the support 81, and prevents shear between the support 81 and the outer layer 82. Moreover, cells which physically couple with the outer layer 82 first, also couple chemically with the outer layer 82 eventually like the support.

Since the medical material 80 composed as thus has sufficient strength, it does not easily become out of alignment, nor break, even if it is located in a living body. Hence, this medical material is preferable to be used particularly as an artificial tooth root, an artificial bone, or prosthetic appliance for bone. Since the whole of this medical material 80 is composed of bioabsorbable ceramics, after inducing osteoblasts etc, it is completely replaced by the induced bone.
Moreover, since the outer layer of the medical material 80 is high in the porosity than the support, as described above, it induces the cells infiltrated into the outer layer, toward the direction of the support. Hence, cells can be early induced into the medical material 80. Further, the replacement of the outer layer with osteoblasts, the infiltration of osteoblasts into the support, and the replacement of the support with osteoblasts are sequentially performed. The medical material 80 can keep high strength averagingly without causing shear between the support 81 and the outer layer 82 until it is replaced with the bone.

As the bioabsorbable ceramics used for this medical material, α-tricalcium phosphate, β - tricalcium phosphate etc. can be cited. In this case, the same kinds and different kinds may be used for the support and the outer layer.
Moreover, on the surface of the support 81 and the outer layer 82, physiologically active substance or auxiliary agent of physiological activity which activates living cells may be coated. As such physiologically active substance or auxiliary agent of physiological activity, cell growth promoting factor, cytokine, antibiotics, cell growth controlling factor, enzyme, protein, polysaccharide, phosphatide, lipoprotein, and mucopolysaccharide can be cited. By these, induction of cells can be promoted.

This medical material is not manufactured with single ceramics material as performed conventionally, but is manufactured with the complex of a plurality of kinds of ceramics material and unsintered calcium phosphate.

As shown in Figure 8c, when the medical material 80 is used for an artificial tooth root, the outer layer 82 is provided in the periphery excluding the upper surface of the support 81, then it is inserted into a jaw bone (alveolar bone). Thereby, osteoblasts infiltrate from the outer layer 82, and the osteoblasts expand in the medical material 80. The abutment 84 in which an artificial tooth root 83 is attached to the upper surface of the support, is inserted after the medical material 80 and the osteoblasts are coupled sufficiently. Then, when the medical material 80 is replaced with an autogenous bone, the autogenous bone directly supports the abutment 84.

In this case, it is preferable that the overall diameter of the medical material 80 is 3-30 mm, particularly 4-20 mm, that the diameter of the support 81 is 2-25 mm, particularly 2-20 mm, and that the thickness of the outer layer is 0.5-10 mm, particularly 1-5mm. And, it is preferable that the diameter of the support 81 is 5-30 %, particularly 10-20 % to the overall diameter of the medical material 80, because it can give more strength to the whole of the medical material 80.

As shown in Figure 8d, when the medical material 80 is used as an artificial bone, the support is formed so as to be the outer shape of the bone or tube shape, and the outer layer 82 is formed on the upper and lower surface of the support. Then, this medical material (artificial bone) 80 is inserted between a bone B1 and a bone B2 to be joined so that the outer layer 82 contacts the bone B1 and the bone B2. Thereby, osteoblasts are induced from the outer layer 82 to couple with the outer layer 82, expand to the support 81, and couple with the support 81. Hence, early healing becomes possible, and the osteoblasts and the medical material bonds firmly.

In this case, though being different according to the portion where the bone is used, the size of the medical material is preferable that the overall height is 1-50 mm, particularly 2-25 mm, that the height of the support 81 is 1-49 mm, particularly 1-20 mm, and that the thickness of the outer layer is 0.5-10 mm, particularly 1-5mm. And, that in which the height of the support 81 is 5-30 %, particularly 10-20 % to the overall height of the medical material 80 is preferable because it gives sufficient strength as a whole.

Next, the medical material 85 of Figure 8b comprises a support 86 composed of non-bioabsorbable ceramics of which the porosity is 0.1-10% (low porosity), particularly 1-5 %, and an outer layer 87 composed of non-bioabsorbable ceramics of which the porosity is 11-80 % (high porosity), particularly 50-70 % formed in the periphery thereof. The difference of the porosity of the support 86 and the outer layer 87 is 11-80 points. The medical material 85 is that in which the material of the medical material 80 of Figure 8a is made to be non-bioabsorbable.

Since, this support 86 has porosity of 0.1-10 % (low) same as the support 81 of Figure 8a, extracellular matrix components can be induced into the support, and the strength as a whole of the medical material 85 can be obtained. Further, by forming the diameter of the support 86 so as to be 5-30 %, particularly 10-20 % of the overall diameter. Particularly, the strength as a whole can be kept, making it possible to comparatively shorten the induction period to whole of the support.

Moreover, the outer layer 87 has the porosity of 11-80 % (high) particularly 50-70 % same as the outer layer 82 of Figure 8a, and the difference of the porosity to that of the support is 10 or more. Further, the diameter of its pore is 50-500 µm or more, particularly 300-400 µm. Hence, it is equipped with a geometrical structure which cells prefer, it induces cells positively. Further, since the outer layer 87 is formed so that the porosity is 10-80 points larger than that of the support 86, shear between the support 86 and the outer layer 87 can be prevented, and cells can be induced into the medical material 85 smoothly.

Since the medical material 85 constituted as described above is not absorbed by a living body after surgical treatment and couples or integrates with a living body, the strength is high right after the surgical treatment (right after the implantation of the medical material). Hence, it is preferably used for those persons who are comparatively late or difficult in inducing cells such as aged persons, and those who are with osteoporosis, osteomalacia. However, even if it is non-bioabsorbable ceramics, it is absorbed and replaced with time of years. Further, since the medical material 85 is composed with two layers whose difference of the porosity is 10-80 points, shears between the support 86 and the outer layer 87 is difficult to occur same as the medial material 80 of Figure 8, and the combining strength of the medical material and a living body can be obtained.

As the non-bioabsorbable ceramics used for this medical material 85, hydroxyapatite, alumina, zirconia, carbon, calcium phosphate, crystallized glass etc. can be cited. In this case, for the support 86 and the outer layer 87, those which are the same kinds may be used, moreover, those which are different kinds may be used. Further, on the surface of the support 86 and the outer layer 87, same as the medical material of Figure 8, physiologically active substance or physiologically active auxiliary agent etc. may be coated.

When this medical substance 85 is used as an artificial tooth root, it is preferable that the overall diameter is made to be 3-8 mm, particularly 3.5-6 mm, the diameter of the support 86 is made to be 3-5 mm, particularly 3-4 mm, the thickness of the outer layer 87 is made to be 02.-2 mm, particularly 0.5-1mm. And, the diameter of the support 86 is preferable to be 5-20 %, particularly 10-15 % to the overall diameter of the medical material 85.
When this medical material 85 is used as an artificial bone, it is preferable that the overall height is made to be 10-200 mm, particularly 10- 150mm. And the height of the support 86 is made to be 100% to the overall height of the medical material 85.

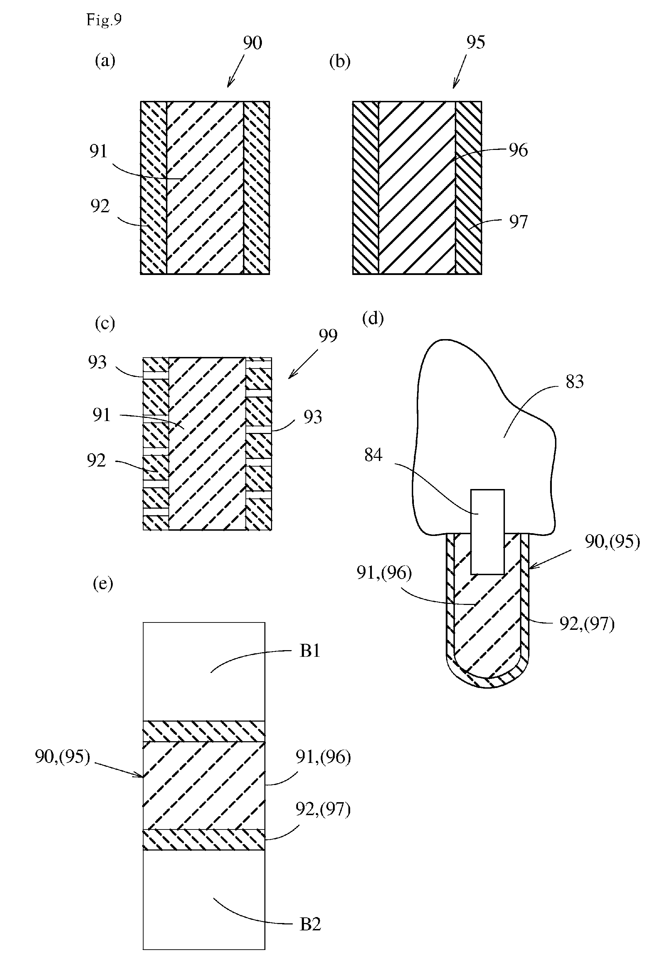

The medical material 90 shown in Figure 9a comprises a support 91 composed of bioabsorbale ceramics of which the porosity is 11-80 % (high porosity), particularly 50-70 %, and an outer layer 92 composed of bioabsorbable ceramics formed in the periphery thereof. The porosity of the outer layer is preferably to be 0.1-10 % (low porosity), particularly 1-5 %. Stated differently, it is that in which the porosity of the support 81 and the outer layer 82 of the medical material 80 of Figure 8 is reversed.

Since the porosity of the support 91 is 11-80 % (high), particularly 50-70 %, it has a geometrical structure which cells prefer, and it induces cells positively. The diameter of the pore formed in the support 91 is 5-500 µm particularly 300-400 µm, which is preferable for osteoblasts to infiltrate.
Since the porosity of this outer layer 92 is 0.1- 10 % (low), particularly 1-5 %, cells can be induced into the outer layer, and the strength as a whole of medical material 90 can be given. Moreover, the difference of the porosity of the outer layer 92 and the support 91 is 10-80 points, shear between the support 91 and the outer layer 92 can be prevented. The diameter of the pore formed in the outer layer 92 is 0.01-10 µm, particularly 0.1- 5 µm. Further by forming the thickness of the outer layer 92 so as to be 5-20 %, particularly 10-15 % of the overall diameter, the strength as a whole can be kept, and the cell induction period to the whole of the medical material can be shortened.
As such bioabsorbable ceramics, same as the medical material 80 of Figure 8, α-tricalcium phosphate, β-tricalcium phosphate etc. can be cited. In this case, as the support and the outer layer, those which are the same kinds can be used, but those which are the different kinds can also be used.

Since the medical material 90 composed as thus has sufficient strength, it does not get out of position easily, even if it made to be located in a living body. Since whole of the medical material 90 is composed of bioabsorbable ceramics, the induced osteoblasts are completely replaced by a bone then. Since this medical material is provided with a layer of low porosity in the outer layer 92, the cell induction period becomes longer than that of the medical material 80 of Figure 8. However, since the combining strength with cells of the support 91 is higher than that of the outer layer 92, once it induces cells, the anchor effect is higher than that of the medical material 80. Moreover, the medical material 90 same as the medical material 80 of Figure 8, can keep high strength averagely without causing shear etc. between the support 91 and the outer layer 92. Hence, in consideration of the suitable cell induction period and the strength, the thickness of the outer layer 92 is preferable to be 5-20 %, particularly 10-15 % to the overall diameter of the medical material 90.
And, as the medical material 99 of Figure 9c, a through hole 93 penetrating the outside and the support 91 may be provided in the outer layer 92. By providing such through hole 93, the cell induction period can be shortened. The diameter of such through hole is preferable to be 0.1-5 mm, particularly 1-3 mm.

When this medical material 90 (99) is used as an artificial tooth root, it is preferable that the overall diameter is made to be 3-20 mm, particularly 4-20 mm, and that the diameter of the support 91 is made to be 2-25 mm, particularly 2-20mm. The thickness of the outer layer 92 is made to be 5-30%, particularly 10-20% to the overall diameter of the medical material 90, because the strength as a whole can be obtained, which is preferable.
When this medical substance 90 (99) is used as an artificial bone, it is preferable that the overall height is made to be 1-50 mm, particularly 2-25 mm, the height of the support 91 to be 1-49 mm, particularly 1-20mm, the thickness of the outer layer is made to be 0.5-10 mm particularly 1-5 mm. And, by forming the thickness of the outer layer so as to be 5-30 %, particularly 10-20 % to the overall height of the medical material 90, the strength as a whole can be obtained, which is preferable.

The medical material 95 shown in Figure 9b comprises a support 96 composed of non-bioabsorbable ceramics of which the porosity is 11-80 %, particularly 50-70 %, and an outer layer 97 formed in the periphery thereof composed of non-bioabsorbable ceramics of which the porosity is 0.1-10 % (low porosity) particularly 1-5 %. Stated differently, it is that in which the porosity of the support 86 and the outer layer 87 of the medical material 85 of Figure 8b is reversed.

Since the porosity of the support 96 is 11-80 % (high), particularly 50-70 %, it has a geometrical structure which cells prefer, and it induces cells positively. The diameter of the pore formed in the support 96 is 5-500 µm, particularly 300-400 µm, which is preferable for osteoblasts to infiltrate.
Since the porosity of this outer layer 97 is 0.1- 10 % (low), cells can be induced into the outer layer, and the strength as a whole medical material 95 can be obtained. Moreover, the difference of the porosity of the outer layer 96 and the support 97 is 10-80 points, shear between the support 96 and the outer layer 97 can be prevented. The diameter of the pore formed in the outer layer 97 is 0.1-10 µm, particularly 0.1- 5 µm. Further by forming the thickness of the outer layer 97 so as to be 5-20 %, particularly 10-15 % of the whole diameter, the strength as a whole can be obtained, and the cell induction period to the whole of the medical material can be comparatively shortened. And, a through hole 93 may be provided in the outer layer 97 as shown in Figure 9c.
As such non-bioabsorbable ceramics, hydroxyapatite, alumina, zirconia, calcium phosphate crystalline body etc. can used like the medical material 85 of Figure 8b. In this case, same kind of material may be used as the support and the outer layer, but those which are the different kinds may also be used.

When this medical material 95 is used as an artificial tooth root (see Figure 9d), it is preferable that the overall diameter is made to be 3-20 mm, particularly 4-20 mm, the diameter of the support 96 is made to be 2-25 mm, particularly 2-20 mm, the thickness of the outer layer is made to be 0.5-10 mm particularly 1-5 mm. And, by forming the thickness of the outer layer so as to be 5-30 %, particularly 10-20 % to the overall height of the medical material 95, the strength as a whole can be obtained, which is preferable.
When this medical material 95 is used as an artificial bone (see Figure 9e), it is preferable that the overall height is made to be 1-50 mm, particularly 2-25 mm, the height of the support 96 is made to be 1-49 mm, particularly 1-20mm, the thickness of the outer layer is made to be 0.5-10 mm particularly 1-5 mm. And, by forming the thickness of the outer layer so as to be 5-30 %, particularly 10-20 % to the overall height of the medical material 95, the strength as a whole can be obtained, which is preferable.

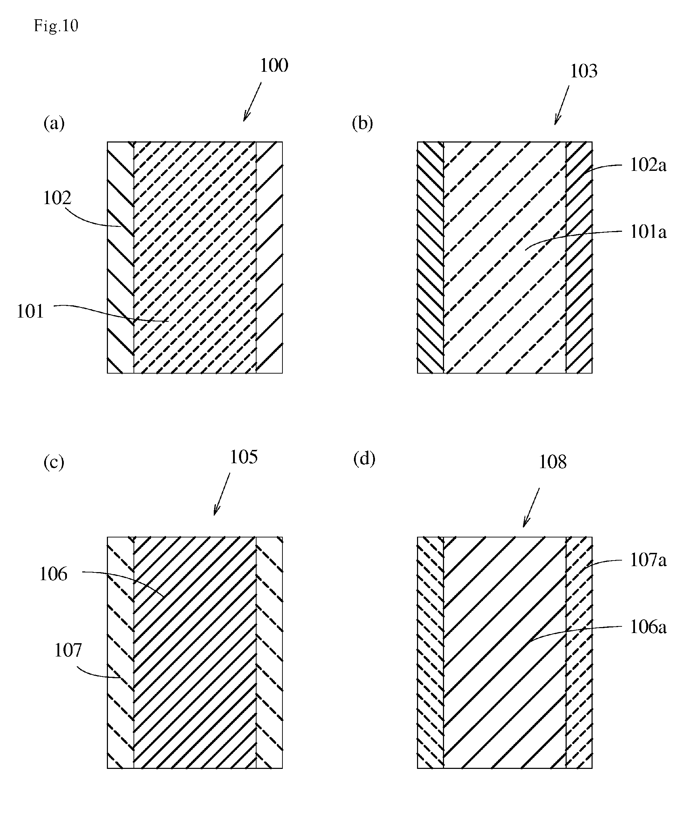

The medical material 100 shown in Figure 10a comprises a support 101 composed of bioabsorbale ceramics of which the porosity is 0.1-10 %, (low porosity), particularly 1-5 %, and an outer layer 102 formed in the periphery thereof composed of non-bioabsorbable ceramics of which the porosity is 11-80 % (high porosity), particularly 50-70 %. The difference of the porosity of the support 101 and the outer layer 102 is 10-80 points.

The support 101 is substantially same as the support 81 of the medical material of Figure 8a. It gives the strength as a whole of the medical material, while inducing cells into the support. And after cells are induced, they are absorbed into the living body.
On the other hand, the outer layer 102 is substantially same as the outer layer 87 of Figure 8b. It is provided with a geometrical structure which cells prefer, and it induces cells positively. And after being induced, they will not be absorbed by the living body for a long period. Hence, it can be used for those persons who are relatively slow or difficult in inducing cells.

Moreover, as the medical material 103 of Figure 10b, the support 101a may be formed from bioabsorbale ceramics of which the porosity is 11-80 % (high porosity), particularly 50-70 %, which is substantially same as the support 91 of the medical material of Figure 9a. And the outer layer 102a may be formed so that the difference of the porosity to the support 101a is 10-80 points from non-bioabsorbable ceramics of which the porosity is 0.1-10 % (low porosity), particularly 1-5 %, which is substantially same as the outer layer 97 of the medical material of Figure 9b.

The medical material 105 shown in Figure 10c comprises a support 106 composed of non-bioabsorbable ceramics of which the porosity is 0.1-10 %, (low porosity), particularly 1-5 %, and an outer layer 107 formed in the periphery thereof composed of bioabsorbable ceramics of the porosity is 11-80 % (high porosity), particularly 50-70 %. The difference of the porosity of the support 106 and the outer layer 107 is 10-80 points.

The support 106 is substantially same as the support 81 of the medical material of Figure 8a. It gives the strength as a whole of the medical material, and it induces cells into the support. And, it is not absorbed into the living body for a long period.
On the other hand, the outer layer 107 is substantially same as the outer layer 82 of Figure 8a. It is provided with a geometrical structure which cells prefer, and it induces cells positively. And it absorbed into the living body.

And, as the medical material 108 of Figure 10d, the support 106a may be formed from non-bioabsorbable ceramics of which the porosity is 0.1-10 % (low porosity), particularly 1-5 %, which is substantially same as the support 86 of the medical material of Figure 9b. And the outer layer 107a may be formed so that the difference of the porosity to the support 106a is 10-80 points from bioabsorbable ceramics of which porosity is 11-80 % (high porosity), particularly 50-70 %, which is substantially same as the outer layer 92 of the medical material of Figure 9a.

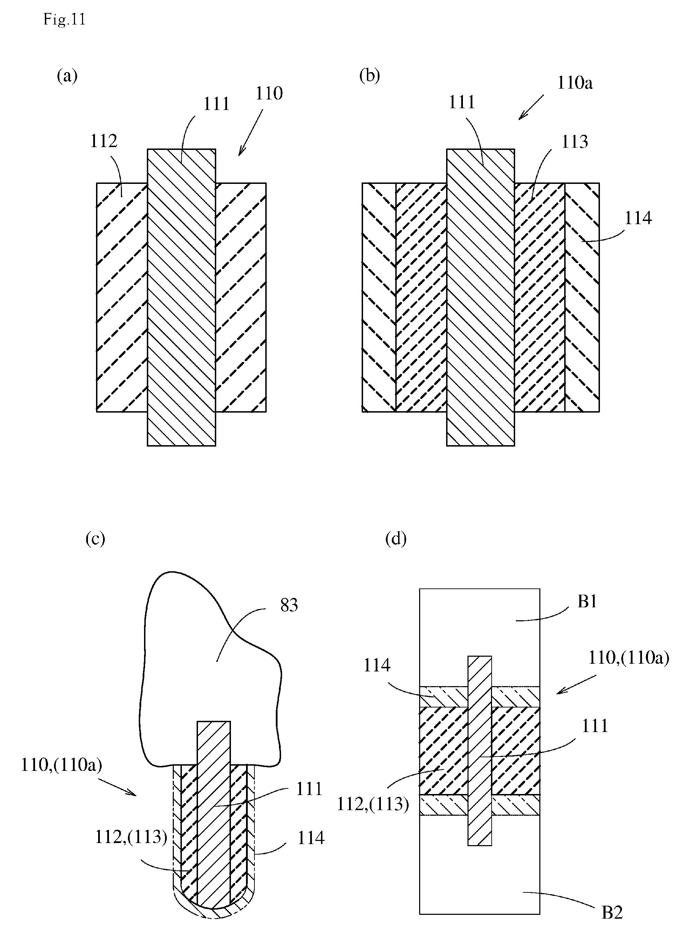

The medical material 110 shown in Figure 11a comprises a tube shape or column shape metal rod 111 and an outer layer 112 composed of bioabsorbable ceramics provided in the periphery thereof.

The metal rod 111 is composed of biocompatible metal such as stainless or titanium. In the case that titanium is used, it is particularly preferable because the examination by MRI under medical treatment is possible. By providing the metal rod 111 as thus, the strength of the medical material can be heightened. Moreover, even when osteoblasts are not sufficiently induced, or even when the density of bone is low after osteoblasts are induced, sufficient strength can be kept. And, when repeat surgery is necessary, the metal rod 111 can be easily removed.

The porosity of the outer layer 112 is 11-80 %, particularly 50-70 %, the diameter of the pore is 50-500 µm, particularly, 300-400 µm. This geometrical structure is that which cells most prefer, the cells will be induced in the outer layer 112 positively. As such ceramics of the outer layer 112, α-tricalcium phosphate, β-tricalcium phosphate etc. can be cited.

The medical material 110 induces cells into outer layer 112 and the metal rod 111 keeps the whole strength. And, since the metal rod 111 is used, the strength can be kept high, allowing the use for whom inducing cells is relatively difficult such as an aged person, or for a portion where inducing cells is relatively difficult such as the portion of osteoporosis, osteomalacia.

As the outer layer for the medical material 110, bioabsorbable ceramics of which the porosity is 11-80 %, particularly 50-70%, of which the diameter of the pore is 5-500 µm, particularly 300-400 µm may be used. In this case also, cells are positively induced into the outer layer because of the geometrical structure. As such non-bioabsorbable ceramics, hydroxyapatite etc. can be cited.

The medical material 110a shown in Figure 120b has the inner layer 13 provided in the periphery of the metal rod 111, and the outer layer 114 formed in the periphery of the inner layer. The inner layer is composed of bioabsorbable ceramics of which the porosity is 0.1-10 % (low porosity), particularly 1-5 %. The outer layer 114 is composed of bioabsorbable ceramics of which the porosity is 11-80 % (high porosity), particularly 50-70 %. In this case, the difference of the porosity of the inner layer 113 and the outer layer 114 is formed so as to be 10-80 points. By providing the metal rod as thus, the strength as a whole of the medical material is further heightened.
The medical material 110a of Figure 11b is medical material where the metal rod 111 is inserted into the support 81 of the medical material 80 of Figure 8a. But the metal rod may be inserted into the medical material 85 of Figure 8b, the medical material 90, 95, 99 of Figure 9a-Figure 9c, or the medical material 100, 103, 105, 108 of Figure 10a-Figure 10d.
Moreover, the medical material 110 (medical material 110a (imaginary line)) can be used for an artificial tooth root (see Figure 11c) and for an artificial bone (see Figure 11) similar to the other medical material of this invention.

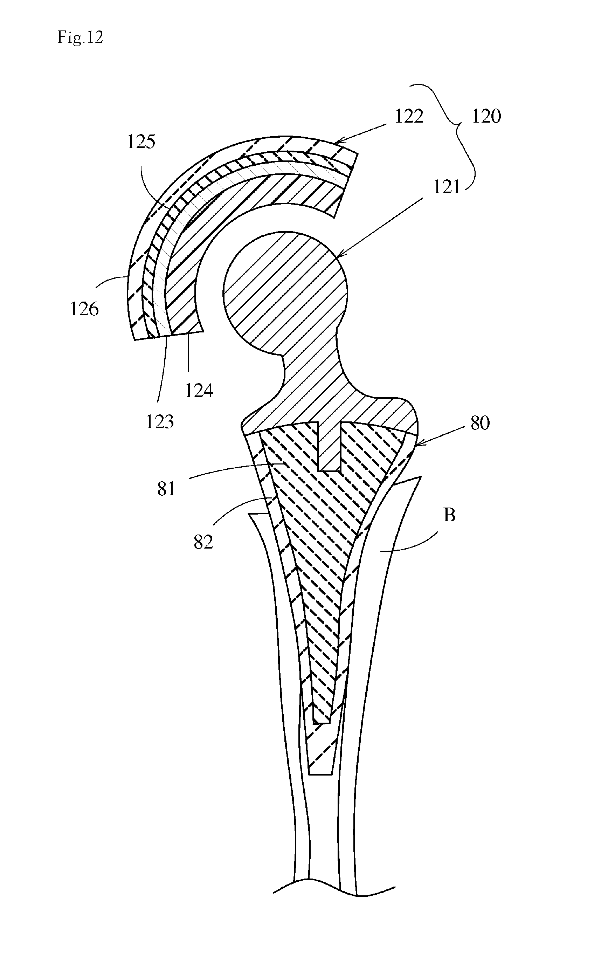

The medical material of Figure 12a is used for an artificial joint. This artificial joint 120 comprises an artificial osteomere 121 and a tray 122. As the artificial osteomere 121, titanium or titanium alloy may be used. As shown in the figure this artificial osteomere 121 is provided on the upper end of the medical material 70 of Figure 8 which is used as an artificial bone attached to the bone B.

The tray 122 of the artificial joint comprises a fan-shaped or saucer-shaped metal substrate 123, a synthetic resin layer 124 provided on the inner surface of the metal substrate, a first layer 125 composed of bioabsorbable ceramics of the porosity 1-10% (low porosity) provided on the outer surface of the metal substrate, and a second layer 126 composed of bioabsorbable ceramics of the porosity 10-80 % (high) provided on the outer surface of the first layer. The difference of the porosity of the first layer and the second layer is 10-70 points.

As the metal substrate 123, stainless or titanium which is high in bio-compatibility may be used. As the synthetic resin layer, synthetic resin such as high polymer polyethylene is preferable giving consideration to the slippage to the artificial osteomere 121 and durability. This thickness is preferable to be 2-20mm, particularly 5-10 mm.

As the first layer 125, that in which the porosity is 0.1-10 %, particularly 1-5 %, the diameter of the pore is 0.01-5 µm, particularly 0.1- 2 µm is used. The thickness of which is 2-10mm, particularly 2-5mm. As the second layer 126, that in which the porosity is 11-80 %, (high), particularly 50-70 %, the diameter of the pore is 50-500 µm, particularly 300-400 µm is used. The thickness of which is 2-10 mm, particularly 2-5 mm.

By implanting the tray 122 of the fartificial joint 122 composed as thus into a living body, induced osteoblasts combine with the second layer 126, and are induced to the first layer 125 through the second layer 126. Since the difference of the porosity to be 10-70 points between the first layer and the second layer 126 is provided, shear is hard to occur. Moreover, as the first layer and the second layer, non-bioabsorbable ceramics may be used, further, as the first layer, bioabsorbable ceramics or non-bioabsorbable ceramics of the porosity 1-5 % (low) may be used, as the second layer, non-bioabsorbable ceramics or bioabsorbable ceramics of the porosity 50-70% (high) may be used.

As the bioabsorbable ceramics, α -tricalcium phosphate, β -tricalcium phosphate, tetracalcium phosphate can be cited. As the non-bioabsorbable, ceramics, hydroxyapatite, alumina, zirconia, carbon, calcium phosphate, crystallized glass etc. can be cited.

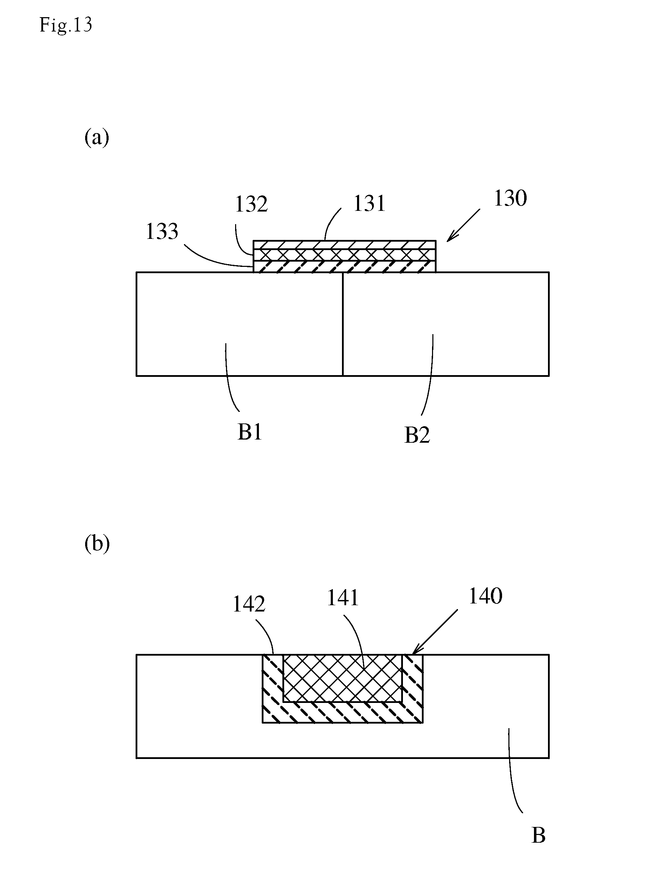

The medical material shown in Figure 13 is used as a bone plate 130. This bone plate comprises a metal substrate 131, a first layer 132 composed of bioabsorbable ceramics of the porosity 0.1-10% (low) provided on its inner surface, and a second layer 133 composed of bioabsorbable ceramics of the porosity 11-80 % (high) provided on the inner surface of the first layer 132. The difference of the porosity of the first layer 132 and the second layer 133 is 10-80 points. As the material for the metal substrate 131, titanium, titanium alloy, or stainless whose bio-compatibility is high can be cited, the thickness of which is 1-10 mm, particularly 2-5 mm.

As the first layer 132, the porosity is 0.1-10 % (low), particularly 1-5 %, the diameter of the pore is 0.01-0.1 µm, particularly 0.1- 1 µm may be used. The thickness of which is 1-5 mm, particularly 1-3 mm.
As the second layer 133, the porosity is 11-80 %, (high), particularly 50-70 %, the diameter of the pore is 50-500 µm, particularly 300-400 µm may be used. The thickness of which is 1-10 mm, particularly 2-5 mm.

The bone plate 130 is bond to the bone B1 and B2 by a link such as a screw. The osteoblasts are induced in the bone plate 130, then couple with the second layer 133, and further couple with the first layer 132. Therefore the bone B1 and the bone B2 are joint mutually. And finally, osteoblasts grow between the bone B1 and the bone B2 mutually, becoming a continuous bone.

In this also, non-bioabsorbable ceramics may be used as the first layer 132, and the second layer 133. Further, as the first layer, bioabsorbable or non-bioabsorbable ceramics of the porosity 0.1-10% (low) may be used. And as the second layer, non-bioabsorbable ceramics or bioabsorbable ceramics of the porosity 11-80% may be used.

The medical material 140 of Figure 13b is used as a prosthetic appliance of bone. It comprises a first layer 141 composed of bioabsorbable ceramics of the porosity 0.1-10% and a second layer 142 composed of bioabsorbable ceramics of the porosity 11-80 % provided so as to cover the first layer. The difference of the porosity of the first layer 141 and the second layer 142 is 10-80 points.
By inserting this prosthetic appliance of bone into the portion of defect, osteoblasts are induced from the periphery of the prosthetic appliance of bone couple with the second layer 142. Then, the osteoblasts reach the first layer 71 from periosteum etc. and obsteo blasts couple with the first layer 141.
In this prosthetic appliance of bone also, non-bioabsorbable ceramics may be used as the first layer and the second layer. Further, as the first layer, bioabsorbable or non-bioabsorbable ceramics of the porosity 0.1-10% (low) may be used. As the second layer, non-bioabsorbable ceramics or bioabsorbable ceramics of the porosity 11-80% (high) may be used.

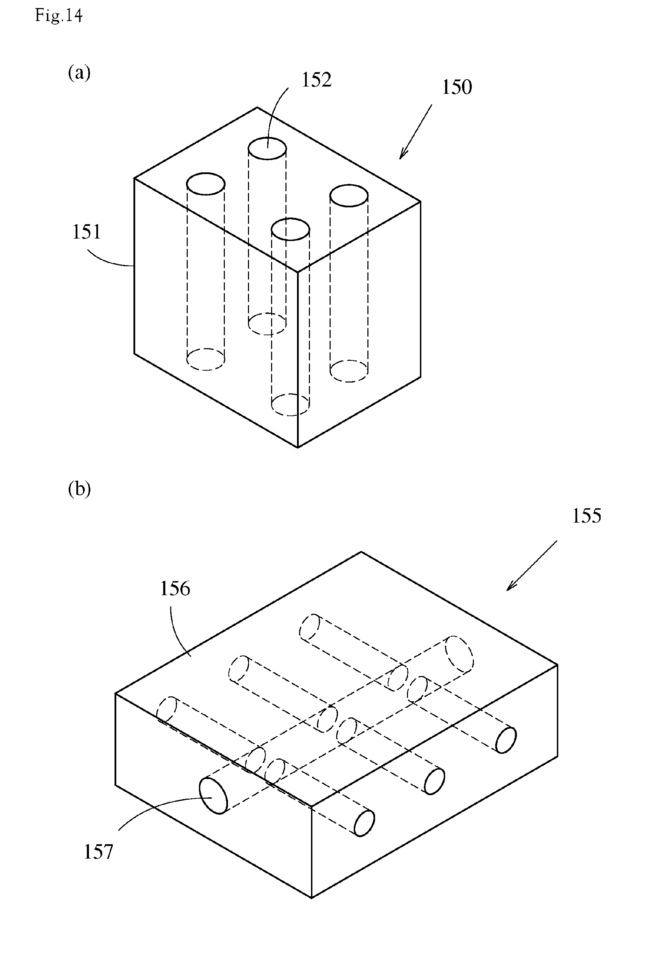

The medical material 150 of Figure 14a comprises a second layer 151 of rectangular parallelepiped shape of which the porosity is 11-80 %, and three first layers 152 having a column shape, of which the porosity is 0.1-10 % formed so as to penetrate the second layer 151. The difference of the porosity of the first layer and the second layer is 10- 80 %. Thus, by providing a plurality of first layers, the whole strength of the medical material 150 is improved. Here, rectangular parallelepiped shape is described as the second layer, but it is not particularly limited to this as far as it is cubical. Moreover, column like is described as the first layer, but it is not limited to cubical shape as far as it is that which is implanted into or penetrates the second layer.

The medical material 155 of Figure 14b comprises a rectangular parallelepiped shaped second layer 156 of which the porosity is 11-80 % and a lattice shape first layer implanted into the second layer 156 of which the porosity is 0.1-10 %. The difference of the porosity of the first layer and the second layer is 10-80 %.

## Claims

1. A medical material, comprising; a metal nonwoven fabric layer composed of a titanium metal fiber whose one side or diameter is 100 µm or less, and a ceramic layer composed of calcium phosphate compound provided adjacently to the metal nonwoven fabric layer.

2. A medical material according to claim 1,
wherein the ceramic layer is composed of bioabsorbable calcium phosphate compound.

3. A medical material according to claim 1,
wherein the ceramic layer is composed of non-bioabsorbable calcium phosphate compound.

4. A medical material according to claim 1,
wherein the metal nonwoven fabric layer is formed to be in a tube shape or a column shape, and the ceramic layer is provided in the periphery of the metal nonwoven fabric layer.

5. A medical material according to claim 1,
wherein the ceramic layer is formed to be in a tube shape or a column shape, and the metal nonwoven fabric layer is provided in the periphery of the ceramic layer.

6. A medical material, comprising; a first layer composed of ceramics of which the porosity is 0.1-10 % (low porosity), and
a second layer provided adjacently to the first layer composed of ceramics of which the porosity is 11-80 % (high porosity),
wherein the difference of the porosity of the first layer and the second layer is 10-80 points.

7. A medical material according to claim 6,
wherein at least two or more of the first layers are equipped.

8. A medical material according to claim 6,
wherein the ceramics of the first layer and the second layer are bioabsorbable ceramics.

9. A medical material according to claim 6,
wherein the ceramics of the first layer and the second layer are non-bioabsorbable ceramics.

10. A medical material according to claim 6,
wherein the ceramics of the first layer is bioabsorbable ceramics or non-bioabsorbable ceramics, and the ceramics of the second layer is non-bioabsorbable ceramics or bioabsorbable ceramics.

11. A medical material according to claim 6,
wherein the first layer is formed to be in a tube shape or a column shape, and the second layer is provided in the periphery of the first layer.

12. A medical material according to claim 6,
wherein the first layer is formed to be in a lattice shape and the second layer is provided so as to fill in the clearance of the lattice.

13. A medical material comprising;
a metal rod having a tube shape or a column shape, and
an outer layer composed of bioabsorbable ceramics or non-bioabsorbable ceramics provided in the periphery of the metal rod.
